# EUROPEAN PATENT SPECIFICATION

(11) **EP 3 020 444 B1**
(45) Date of publication and mention of the grant of the patent: **02.09.2020**
(21) Application number: 14822799.4
(22) Date of filing: 08.07.2014
(51) Int. Cl.: A61M 25/14, A61M 25/01, A61M 25/10

(54) **CATHETER**
KATHETER
CATHÉTER

(30) Priority: 09.07.2013 JP 2013143240
(43) Date of publication of application: 18.05.2016
(73) Proprietor: Goodman Co., Ltd., Aichi 460-0008 (JP)
(72) Inventor: IWANO, Kenshi, Seto-shi Aichi 489-0976 (JP); MIYAKE, Takamasa, Seto-shi Aichi 489-0976 (JP); NAKAJIMA, Megumi, Nagoya-shi Aichi 460-0008 (JP); OGAWA, Tomokazu, Seto-shi Aichi 489-0976 (JP); OGAWA, Keisuke, Seto-shi Aichi 489-0976 (JP); FUJISAWA, Soichiro, Seto-shi Aichi 489-0976 (JP)
(74) Representative: Epping - Hermann - Fischer
(86) International application number: PCT/JP2014/068111
(87) International publication number: WO 2015/005304

(56) References cited:
- EP-A1- 2 495 006
- WO-A1-01/45788
- WO-A1-02/28466
- WO-A1-94/11053
- JP-A- H10 503 386
- JP-A- 2012 228 296
- US-A- 5 810 867
- US-A1- 2012 029 367
- US-A1- 2012 296 367

## Description

### Technical Field

The present invention relates to a catheter.

### Background art

In related art, in treatments such as PTA (percutaneous transluminal angioplasty) and PTCA (percutaneous transluminal coronary angioplasty), a balloon catheter is used. The balloon catheter is provided with a catheter tube and a balloon. The balloon is provided on a distal end side of the catheter tube. A user introduces the balloon into a constricted section or a blocked section of a blood vessel and inflates the balloon, thus expanding the constricted or blocked section. The user is, for example, an operator such as a medical doctor.

The catheter tube of the balloon catheter is provided with an outer tube and an inner tube. The inner tube is inserted into the outer tube. The balloon is connected to a distal end portion of the outer tube. The balloon inflates or deflates as a result of a compressed fluid flowing through a lumen of the outer tube. The inner tube is provided in a state in which a leading end portion of the inner tube extends further to the leading end side than a leading end of the outer tube. The leading end portion of the inner tube that extends from the leading end of the outer tube passes through the interior of the balloon and extends further to the leading end side than the balloon. A lumen of the inner tube is a guide wire lumen, through which a guide wire can be inserted. When the user introduces the balloon catheter into a human body, the guide wire that is introduced into the human body in advance is inserted through the lumen of the inner tube. The user introduces the balloon catheter into the human body in a state in which the guide wire is inserted through the lumen of the inner tube.

As a manner of inserting the guide wire, so-called an RX type is known. The RX type is a type in which the guide wire can be led to the outside from a midway position in the axial line direction of the outer tube. In the RX type balloon catheter, a guide wire port, which penetrates a peripheral wall portion of the outer tube, is formed in a midway position in the axial line direction of the outer tube. The lumen of the inner tube is open to the outside of the catheter via the guide wire port. In this case, the guide wire that is introduced inside the inner tube from the leading end portion of the inner tube is introduced to the outside of the catheter via the guide wire port.

In the meantime, in this type of the balloon catheter, with the object of increasing rigidity etc., a core wire is sometimes provided (refer to Patent Literature 1, for example). The core wire is provided by being inserted into the lumen of the outer tube. The core wire is disposed across the guide wire port in the axial line direction, for example. In this case, the inner tube is provided in an area further to the leading end side than the guide wire port in the lumen of the outer tube. Thus, in this leading end side area, the core wire is inserted between an outer peripheral surface of the inner tube and an inner peripheral surface of the outer tube. As a result, it is necessary for a section of the core wire disposed in the leading end side area to be formed with a narrow diameter.

On the other hand, it is preferable that a section of the core wire further to a base end side than the section formed with the narrow diameter be formed such that an outer diameter becomes gradually larger from the leading end side toward the base end side. It is therefore conceivable that the core wire be formed, for example, such that the outer diameter becomes slowly larger at a constant rate from the leading end side toward the base end side. In this case, the rigidity of the core wire can be gradually reduced from the base end side to the leading end side, and it is possible to improve a kink resistance performance.

### Citation List

### Patent Literature

Patent Literature 1: Japanese Laid-Open Patent Publication No. 2008-200317. See as well EP2495006 A1, US2012296367 A1 and WO0145788 A1.

### Summary of Invention

### Problems that invention is to solve

In the meantime, in the above-described balloon catheter, in the state in which the guide wire is inserted through the lumen of the inner tube, since the guide wire is present, the rigidity increases in the area further to the leading end side than the guide wire port through which the guide wire is inserted. On the contrary, the guide wire is not present in an area further to the base end side than the guide wire port. Thus, rigidity in the area further to the base end side than the guide wire port does not change from a state before the guide wire is inserted into the catheter. In this case, rigidity changes locally at the location of formation of the guide wire port in the axial line direction. Therefore, when the user introduces the balloon catheter into the human body along the guide wire, in this location, there is a possibility that a pressing force from the base end side is not properly transmitted to the leading end side. As a result, it is possible that operability may deteriorate.

Further, in the above-described structure in which the core wire is disposed across the guide wire port in the axial line direction, rigidity is imparted in the vicinity on a base end side of the guide wire port by the core wire. As a result, an effect can be anticipated that the local change in rigidity at the location of formation of the guide wire port is suppressed. However, the outer diameter of the above-described core wire becomes gradually larger at a constant rate from a narrow diameter area disposed further to the leading end side than the guide wire port toward the base end side. It is thus difficult to obtain a sufficient size of the outer diameter in the vicinity on the base end side of the guide wire port. As a result, it is conceivable that it is difficult to impart sufficient rigidity in the vicinity on the base end side. Therefore, in the above-described core wire, the above-described problem that the pressing force is not properly transmitted to the leading end side when the catheter is introduced into the human body is still not resolved, and an improvement is demanded.

In light of the above-described circumstances, it is a main object of the present invention to provide a catheter capable of achieving improved operability when introducing the catheter into a human body.

### Solution to problems

The invention is defined by the appended claims. Subject-matter referred to as embodiments and/or inventions not being under the scope of the appended claims merely represent possible exemplary executions and are not part of the invention. In order to solve the above-described problem, a catheter of a first aspect of the invention is characterized by including: a tube having a lumen in an interior thereof; a guide wire port formed in a midway position in an axial line direction of the tube, the guide wire port penetrating a peripheral wall portion surrounding the lumen; and a core wire inserted into the lumen and extending across the guide wire port in the axial line direction. The core wire includes a leading end side area, which is an area positioned further to a leading end side than the guide wire port, and a tapered area, which is an area further to a base end side than the leading end side area and which is formed such that a cross-sectional area thereof in a direction orthogonal to the axial line direction becomes continuously larger from the leading end side toward the base end side. The tapered area includes a first tapered area, which is an area extending from a base end of the leading end side area toward the base end side, and which is an area including a same position as the guide wire port in the axial line direction, and a second tapered area, which is an area extending from a base end of the first tapered area toward the base end side. A rate of increase of the cross-sectional area from the leading end side toward the base end side in the first tapered area is larger than a rate of increase of the cross-sectional area from the leading end side toward the base end side in the second tapered area.

According to the present invention, the core wire is provided with the leading end side area that is provided further to the leading end side than the guide wire port, and with the tapered area that is provided further to the base end side than a leading end area. The tapered area has a first tapered area, which is an area including the same position as the guide wire port in the axial line direction, and a second tapered area, which is an area further to the base end side than the first tapered area. A rate of increase of the cross-sectional area from the leading end side toward the base end side is larger in the first tapered area than in the second tapered area. As a result, in comparison to a configuration in which the cross-sectional area of the tapered area increases at a constant rate from the leading end side toward the base end side, it is possible to enlarge the cross-sectional area of the core wire in a position in the vicinity on the base end side of the guide wire port in the axial line direction. It is thus possible to increase the rigidity of the core wire. Accordingly, it is possible to improve the transmissibility of a pressing force when the catheter is introduced into a human body. As a result, it is possible to achieve an improvement in operability.

With respect to the catheter of the first aspect of the invention, the catheter of a second aspect of the invention is characterized in that a boundary portion between the first tapered area and the second tapered area is positioned further to the leading end side than a center of the tapered area in the axial line direction.

Under the condition that the cross-sectional area of the boundary portion between the first tapered area and the second tapered area is the same, when a comparison of magnitudes of the rate of increase of the cross-sectional area from the leading end side to the base end side in the first tapered area is made for a case in which the boundary portion is positioned to the leading end side in the tapered area, and for a case in which the boundary portion is positioned to the base end side, it is conceivable that the rate of increase of the cross-sectional area is larger in the former case than in the latter case. Here, paying attention to this point, the present invention causes the boundary portion between the first tapered area and the second tapered area to be positioned further to the leading end side than a center position of the tapered area in the axial line direction. In this case, by making the rate of increase of the cross-sectional area larger in the first tapered area, it is possible to enlarge the cross-sectional area of the core wire in the position in the vicinity on the base end side of the guide wire port in the axial line direction. It is thus possible to further increase the rigidity of the core wire. As a result, it is possible to further improve the transmissibility of the pressing force when the catheter is introduced into the human body.

With respect to the catheter of the second aspect of the invention, the catheter of a third aspect of the invention is characterized in that the boundary portion is positioned further to the leading end side than a location positioned to the base end side, from a leading end portion of the tapered area, by a distance of 1/4 of a total length of the tapered area.

According to the present invention, the boundary portion between the first tapered area and the second tapered area is positioned further to the leading end side than the location positioned to the base end side, from the leading end portion of the tapered area, by the distance of 1/4 of the total length of the tapered area. Accordingly, it is possible to make the rate of increase from the leading end side toward the base end side of the cross-sectional area of the first tapered area even larger than in the second aspect of the invention. In this case, it is possible to even further enlarge the cross-sectional area of the core wire in the position in the vicinity on the base end side of the guide wire port in the axial line direction. It is thus possible to even further increase the rigidity of the core wire. As a result, it is possible to even further improve the transmissibility of the pressing force when the catheter is introduced into the human body.

With respect to the catheter of the second aspect or the third aspect of the invention, the catheter of a fourth aspect of the invention is characterized in that the cross-sectional area of the boundary portion is larger than a mean value of the cross-sectional areas of both ends of the tapered area in the axial line direction.

According to the present invention, with respect to the configuration of the second aspect of the invention in which the boundary portion between the first tapered area and the second tapered area is positioned further to the leading end side than the center position of the tapered area, the cross-sectional area of the boundary portion is larger than the mean value of the cross-sectional areas of both ends of the tapered area in the axial line direction. In this case, in comparison to a configuration in which the cross-sectional area of the boundary portion is equal to or smaller than the mean value of the above-described cross-sectional areas, it is possible to increase the rate at which the cross-sectional area of the first tapered area increases from the leading end side toward the base end side. As a result, it is possible to yet further enlarge the cross-sectional area of the core wire in the position in the vicinity on the base end side of the guide wire port in the axial line direction. It is thus possible to yet further increase the rigidity of the core wire. Accordingly, it is possible to yet further improve the transmissibility of the pressing force when the catheter is introduced into the human body.

With respect to the catheter of any one of the first to fourth aspects of the invention, the catheter of a fifth aspect of the invention is characterized in that the core wire further includes a base end side area, which is an area extending from a base end of the second tapered area toward the base end side. The base end side area is formed to have the cross-sectional area that is constant from the leading end side toward the base end side, or is formed to have the cross-sectional area that becomes larger from the leading end side toward the base end side at a rate of increase that is smaller than the second tapered area. A difference between the rates of increase of the cross-sectional area of the first tapered area and the second tapered area is the same as or substantially the same as a difference between the rates of increase of the cross-sectional area of the second tapered area and the base end side area.

According to the present invention, the difference between the rates of increase of the cross-sectional area of the first tapered area and the second tapered area is the same as or substantially the same as the difference between the rates of increase of the cross-sectional area of the second tapered area and the base end side area. Thus, local changes (amounts) that occur in the rate of increase of the cross-sectional area in the boundary portion between the first tapered area and the second tapered area and the boundary portion between the second tapered area and the base end side area, respectively, can be made the same. In other words, in this case, local changes in the rate of increase of the cross-sectional area occurring respectively in each of the boundary portions can be uniformly distributed to each of the boundary portions. As a result, it is possible to suppress the above-described local changes occurring respectively in each of the boundary portions. It is therefore possible to suppress local changes in rigidity occurring respectively in each of the boundary portions. Accordingly, with the structure provided with the two areas having the different rates of increase of the cross-sectional area in the tapered area, it is possible to suppress a deterioration in a kink resistance performance.

With respect to the catheter of any one of the first to fifth aspects of the invention, the catheter of a sixth aspect of the invention is characterized by including: an outer tube corresponding to the tube, the outer tube internally having a fluid lumen corresponding to the lumen; an inner tube inserted into the fluid lumen in a state in which a part of the inner tube extends further to the leading end side than a leading end of the outer tube, the inner tube having a guide wire lumen through which the guide wire is insertable, and a base end portion of which communicates with the guide wire port; and a balloon, which covers, from the outside, the part of the inner tube extending from the leading end of the outer tube, and a base end portion of which is connected to a leading end portion of the outer tube. The leading end side area of the core wire is inserted into the fluid lumen between an outer peripheral surface of the inner tube and an inner peripheral surface of the outer tube.

The balloon catheter is provided with the outer tube and the inner tube. The inner tube is inserted into the fluid lumen of the outer tube. The fluid flows between the outer peripheral surface of the inner tube and the inner peripheral surface of the outer tube. Thus, in order to suppress a deterioration in flowability of the fluid flowing between the inner tube and the outer tube, it is necessary to make the cross-sectional area of the leading end side area as small as possible. Therefore, there is a possibility that, in the tapered area extending from the leading end side area to the base end side, the cross-sectional area in the position in the vicinity on the base end side of the guide wire port may become smaller. By adopting the balloon catheter of the first aspect of the invention, it is possible to enlarge the cross-sectional area of the core wire in the position in the vicinity on the base end side of the guide wire port. By enlarging the cross-sectional area of the core wire 30 in the position in the vicinity on the base end side of the guide wire port 21, a through-flow performance of the compressed fluid is secured, and it is possible to yet further improve the transmissibility of the pressing force when the balloon catheter 10 is introduced into the human body.

### Brief description of Drawings

Fig. 1 is an overall side view showing a configuration of a balloon catheter.
Fig. 2 is a vertical cross-section view showing a configuration in the vicinity of a guide wire port.
Fig. 3 (a) is a side view showing a configuration of a core wire, and Fig. 3 (b) is an enlarged side view showing an enlarged section of the core wire.

### Description of Embodiments

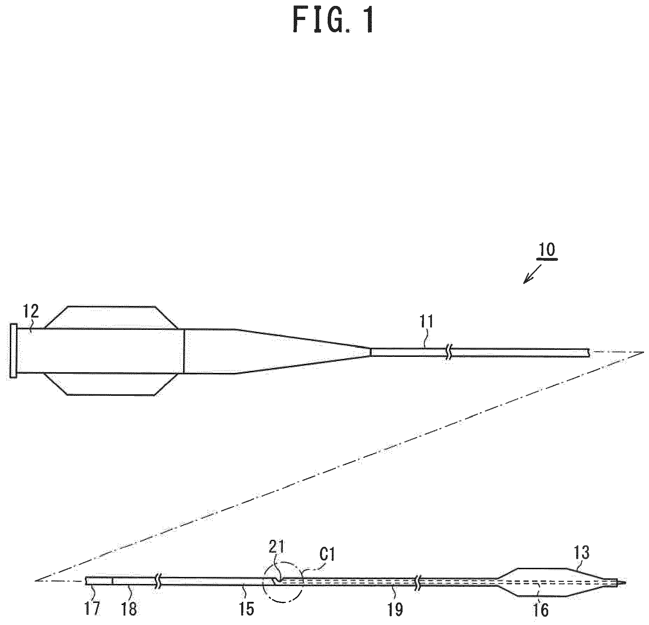

Hereinafter, an embodiment that embodies the present invention will be explained with reference to the drawings. In the present embodiment, a balloon catheter provided with a balloon that can inflate and deflate is embodied. Fig. 1 is an overall side view showing a configuration of the balloon catheter.

As shown in Fig. 1, a balloon catheter 10 is provided with a catheter shaft 11, a hub 12, and a balloon 13. The hub 12 is attached to a base end portion (a proximal end portion) of the catheter shaft 11. The balloon 13 is attached to a leading end side (a distal end side) of the catheter shaft 11.

The catheter shaft 11 includes a plurality of tubular shafts (tubes). The catheter shaft 11 is an inner/outer multiple-tube structure from at least a midway position in an axial line direction (a lengthwise direction) to the position of the balloon 13. Specifically, the catheter shaft 11 is provided with an outer shaft 15 and an inner shaft 16. An inner diameter of the inner shaft 16 is smaller than an inner diameter of the outer shaft 15. An outer diameter of the inner shaft 15 is smaller than an outer diameter of the outer shaft 15. The outer diameter of the inner shaft 16 is smaller than the inner diameter of the outer shaft 15. The inner shaft 16 is inserted into the outer shaft 15. Accordingly, the catheter shaft 11 is configured as an inner/outer double-tube structure.

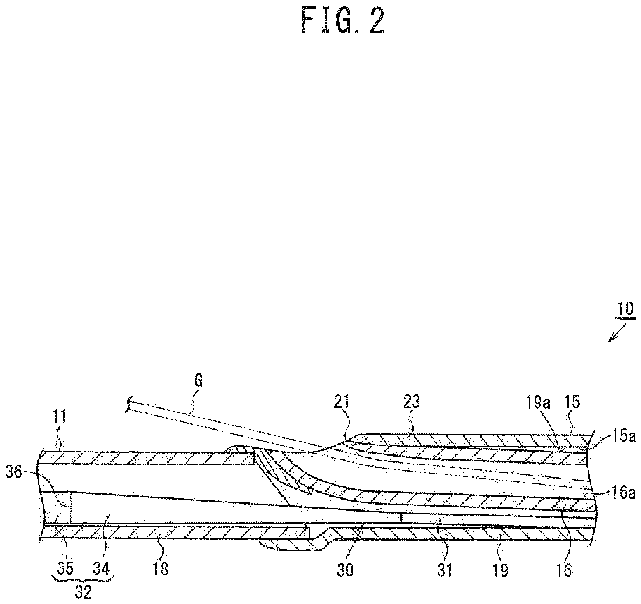

The outer shaft 15 is a tube internally having an outer tube hole 15a. Specifically, the outer shaft 15 is a tube-shaped member having the outer tube hole 15a (refer to FIG. 2) that is open at both ends as well as continuously along the whole of the axial line direction. The outer shaft 15 is provided with a plurality of shafts 17 to 19 aligned in the axial line direction. Each of the plurality of shafts 17 to 19 is connected to the adjacent shaft by welding (thermal welding) or such. However, each of the shafts 17 to 19 need not necessarily be connected by welding, and may be connected by another bonding method, such as adhesion etc.

The respective shafts 17 to 19 are the proximal shaft 17, the mid-shaft 18, and the distal shaft 19, in that order from the base end side. The proximal shaft 17 is formed of a metal, such as an Ni-Ti alloy, stainless steel or the like. A base end portion of the proximal shaft 17 is connected to the hub 12. The mid-shaft 18 is formed of a thermoplastic polyamide elastomer. The rigidity of the mid-shaft 18 is lower than the rigidity of the proximal shaft 17. The distal shaft 19 is formed of a thermoplastic polyamide elastomer. The rigidity of the distal shaft 19 is lower than the rigidity of the mid-shaft 18.

It should be noted that the proximal shaft 17 need not necessarily be formed of a metal, and may be formed of a synthetic resin. Further, the mid-shaft 18 and the distal shaft 19 need not necessarily be formed of the polyamide elastomer and may be formed of another synthetic resin. Furthermore, the rigidity in the present specification refers to a magnitude of a moment operating when it is attempted to bend the catheter in a direction orthogonal to the axial line direction. The inner shaft 16 is a tube-shaped member having an inner tube hole 16a that is open at both ends as well as continuously along the whole of the axial line direction (refer to FIG. 2). The inner tube hole 16a is a tube hole that functions as a guide wire lumen. Thus, a guide wire G can be inserted through the inner tube hole 16a. The inner shaft 16 is inserted into the distal shaft 19 of the outer shaft 15. Abase end portion of the inner shaft 16 is connected to a midway position in the axial line direction of the outer shaft 15. Specifically, the base end portion of the inner shaft 16 is connected to a boundary portion between the mid-shaft 18 and the distal shaft 19.

A guide wire port 21 is formed at a connection section of the outer shaft 15 to the inner shaft 16. Hereinafter, a structure around the guide wire port 21 will be explained with reference to Fig. 2. Note that Fig. 2 is a vertical cross-section view showing the structure in the vicinity of the guide wire port 21. Further, Fig. 2 shows an area C1 shown in Fig. 1.

As shown in Fig. 2, the guide wire port 21 is formed on a base end portion of the distal shaft 19. Internally, the distal shaft 19 has a tube hole 19a that is a part of the outer tube hole 15a. In other words, the outer tube hole 15a includes the tube hole 19a. Of the outer tube hole 19a, the tube hole 19a is a tube hole positioned on the base end side. The guide wire port 21 is formed so that it penetrates through a peripheral wall portion 23 that surrounds the tube hole 19a. In this way, the guide wire port 21 is formed in the midway position in the axial line direction of the outer shaft 15 (refer to FIG. 1) such that the guide wire port 21 penetrates the peripheral wall portion 23 surrounding the tube hole 19a.

The base end portion of the inner shaft 16 is connected to a peripheral edge portion of the guide wire port 21 in the distal shaft 19 (the peripheral wall portion 23). The base end of the inner tube hole 16a of the inner shaft 16 is open to the outside of the catheter 10, via the guide wire port 21. Accordingly, the guide wire G that is introduced into the inner tube hole 16a from a leading end side opening of the inner shaft 16 can be introduced to the outside from the base end of the inner tube hole 16a through the guide wire port 21. In other words, the balloon catheter 10 is a so-called RX type catheter, which is a catheter configured such that the guide wire G can be introduced to the outside at the midway position in the axial line direction.

Apart of the inner shaft 16 extends further to the leading end side than the leading end of the outer shaft 15 (refer to FIG. 1). The balloon 13 is provided such that it covers, from the outside, the inner shaft 16 extending from the leading end of the outer shaft 15. The balloon 13 is formed of a thermoplastic polyamide elastomer. However, the balloon 13 may be formed of another thermoplastic synthetic resin, such as polyethylene, polypropylene or the like.

Abase end portion of the balloon 13 is connected to a leading end portion of the outer shaft 15. In this way, the balloon 13 covers, from the outside, the inner shaft 16 extending from the leading end of the outer shaft 15, and the base end portion of the balloon 13 is connected to the leading end portion of the outer shaft 15. A leading end portion of the balloon 13 is connected to a leading end portion of the inner shaft 16. The interior of the balloon 13 is communicated with the hub 12, via the outer tube hole 15a of the outer shaft 15. A compressed fluid that is supplied via the hub 12 is supplied to the balloon 13 via the outer tube hole 15a. In this case, the outer tube hole 15a functions as a fluid lumen to cause the compressed fluid to flow. When the compressed fluid is supplied to the balloon 13 via the outer tube hole 15a, the balloon 13 is inflated. When a negative pressure is applied to the outer tube hole 15a and the compressed fluid is discharged, the balloon 13 is deflated.

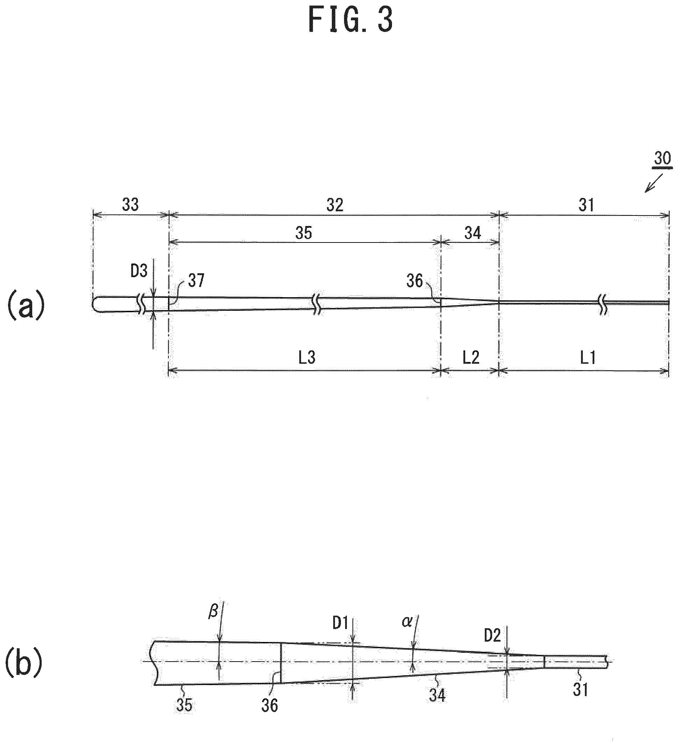

The balloon catheter 10 is provided with a core wire 30. The core wire 30 is provided in the balloon catheter 10 with the object of improving rigidity and the like of the catheter 10. Hereinafter, a configuration of the core wire 30 will be explained with reference to Fig. 2 and Fig. 3. Note that Fig. 3 (a) is a side view showing the configuration of the core wire 30 and Fig. 3 (b) is an enlarged side view showing an enlarged section of the core wire 30.

The core wire 30 is a linear member. The core wire 30 is formed of a metal material, for example. For example, the core wire 30 is formed of stainless steel. A transverse cross-section of the core wire 30 has a circular shape. As shown in Fig. 3 (a), the core wire 30 is formed such that the outer diameter on the leading end side is smaller than the outer diameter on the base end side. As a result, the rigidity of the core wire 30 is reduced the further toward the leading end side from the base end side. Note that the transverse cross-section is a cross-section perpendicular to the axial line direction of the core wire 30.

It should be noted that the core wire 30 may be formed of a material other than stainless steel, and may be formed, for example, of a superelastic alloy, such as a nickel titanium alloy or the like. Further, the transverse cross-section of the core wire 30 need not necessarily be a circular shape and may be another shape, such as a square shape, a hexagonal shape, or the like.

The core wire 30 is provided with a leading end side area 31, a tapered area 32, and a base end side area 33. The leading end side area 31 is an area extending from the leading end toward a base end side of the core wire 30. Note that the leading end side area 31 is an area positioned further to the leading end side than the guide wire port 21. The tapered area 32 is an area provided continuously toward the base end side from the leading end side area 31. In other words, the tapered area 32 is an area extending from a base end of the leading end side area 31 toward the base end side. The base end side area 33 is an area provided continuously toward the base end side from the tapered area 32. In other words, the base end side area 33 is an area extending from a base end of the tapered area 32 toward the base end side. The base end side area 33 is an area including a base end portion of the core wire 30.

Of each of the areas 31 to 33 of the core wire 30, the leading end side area 31 is the area including the section with the smallest outer diameter. The leading end side area 31 is formed in a tapered shape. Therefore, an outer diameter of the leading end area 31 gradually becomes larger from the leading end side toward the base end side. In the present embodiment, a length L1 of the leading end side area 31 in the axial line direction is set to be 80 mm. Note that the leading end side area 31 need not necessarily be formed in the tapered shape. The leading end side area 31 may be a columnar area whose outer diameter is constant across the whole area in the axial line direction.

The tapered area 32 is an area formed in a tapered shape such that an outer diameter continuously becomes larger from the leading end side toward the base end side. In the present embodiment, a length of the tapered area 32 in the axial line direction (L2 + L3) is set to be 130 mm.

An outer diameter of the base end side area 33 is constant across the whole area in the axial line direction. Specifically, in contrast to the leading end side area 31 and the tapered area 32, the base end side area 33 is a non-tapered area. A length of the base end side area 33 in the axial line direction is sufficiently longer than the leading end side area 31 and the taper area 32. In the present embodiment, the length of the base end side area 33 is set to be between 700 and 1500 mm.

Next, the tapered area 32 will be explained in detail.

The tapered area 32 has a first tapered area 34 and a second tapered area 35. The first tapered area 34 is an area provided continuously toward the base end side from the leading end side area 31. In other words, the first tapered area 34 is an area extending from the base end of the leading end side area 31 toward the base end side. The second tapered area 35 is an area provided continuously toward the base end side from the first tapered area 34. In other words, the second tapered area 35 is an area extending from a base end of the first tapered area 34 toward the base end side. In the first tapered area 34, a rate of increase of the outer diameter from the leading end side to the base end side is comparatively larger than that of the second tapered area 35. Thus, as shown in Fig. 3 (b), a first inclination angle α is larger than a second inclination angle β. The first inclination angle α is an inclination of an outer peripheral surface of the first tapered area 34 with respect to the axial line direction. The second inclination angle β is an inclination of an outer peripheral surface of the second tapered area 35 with respect to the axial line direction. Specifically, a rate of increase of the transverse cross-sectional area from the leading end side to the base end side of the first tapered area 34 is larger than a rate of increase of the transverse cross-sectional area from the leading end side to the base end side of the second tapered area 35.

It should be noted that, for the inclination angles α and β of the outer peripheral surface with respect to the axial line direction, an angle on an acute angle side and an angle on an obtuse angle side exist, and here it is assumed that the "inclination angles α and β" of the present specification refer to the angles on the acute angle side. Further, in the present specification, the transverse cross-sectional area of the core wire 30 (of each of the areas 31 to 35) is a cross-sectional area in a direction orthogonal to the axial line direction of the core wire 30.

The rate of increase of the outer diameter from the leading end side to the base end side of the first tapered area 34 is larger than the rate of increase of the outer dimeter from the leading end side to the base end side of the leading end side area 31. Specifically, the rate of increase of the outer diameter (the transverse cross-sectional area) from the leading end side to the base end side is larger in the first tapered area 34 than in either of each of the adjacent areas 31 and 35 on both sides in the axial line direction of the first tapered area 34. Note that, in the present embodiment, in each of the leading end side area 31 and the second tapered area 35, the rate of increase in the outer diameter from the leading end side toward the base end side is the same. In other words, the inclination of the outer peripheral surface with respect to the axial line direction is the same in each of the leading end side area 31 and in the second tapered area 35.

The length L2 of the first tapered area 34 in the axial line direction is shorter than the length L3 of the second tapered area 35 in the axial line direction. Specifically, when a dimension ratio of the length L2 of the first tapered area 34 and the length L3 of the second tapered area 35 is expressed as L2/L3, L2/L3 is smaller than 1 (0 < L2/L3 < 1). Specifically, L2/L3 is smaller than 1/3, and more specifically, is smaller than 1/5.

Expressed differently, a boundary portion 36 between the first tapered area 34 and the second tapered area 35 in the tapered area 32 is positioned further to the leading end side than a center of the tapered area 32 in the axial line direction. Specifically, the boundary portion 36 is positioned further to the leading end side than a location that is positioned to the base end side by a length of 1/4 of the total length (L2 + L3) of the tapered area 32 from the leading end portion of the tapered area 32. More specifically, the boundary portion 36 is positioned further to the leading end side than a location that is positioned to the base end side by a length of 1/6 of the total length (L2 + L3) of the tapered area from the leading end portion of the tapered area 32.

Note that, in the present embodiment, the length L2 of the first tapered area 34 is set to be 20 mm, and the length L3 of the second tapered area 35 is set to be 110 mm. Thus L2/L3 is 2/11.

In addition, in the above-described configuration in which the boundary portion 36 between the first tapered area 34 and the second tapered area 35 is positioned further to the leading end side than a center position of the tapered area 32 in the axial line direction, an outer diameter D1 of the boundary portion 36 is larger than a mean value of an outer diameter D2 of the leading end portion of the tapered area 32 and an outer diameter D3 of the base end portion of the tapered area 32. The outer dimeter D2 is a minimum outer diameter of the tapered area 32. The diameter D3 is a maximum outer diameter of the tapered area 32. Further, a transverse cross-sectional area of the boundary portion 36 is larger than a mean value of a transverse cross-sectional area of the leading end portion of the tapered area 32 and a transverse cross-sectional area of the base end portion of the tapered area 32. In other words, the transverse cross-sectional area of the boundary portion 36 is larger than a mean value of a minimum transverse cross-sectional area of the tapered area 32 and a maximum transverse cross-sectional area of the tapered area 32.

Next, the rate of increase of the transverse cross-sectional area from the leading end side toward the base end side in the first tapered area 34 and the second tapered area 35 will be explained in more detail.

The rate of increase of the transverse cross-sectional area from the leading end side to the base end side in the tapered areas 34 and 35 is expressed as a variation amount of the transverse cross-sectional area per unit length in the axial line direction. Hereinafter, the rate of increase of the transverse cross-sectional area from the leading end side to the base end side in the tapered areas 34 and 35 is also referred to as a transverse cross-section increase ratio. The variation amount of the transverse cross-sectional area per unit length in the axial line direction is an increase amount of the transverse cross-sectional area when seen from the leading end side toward the base end side. The transverse cross-sectional area increase ratio of the tapered area 34 is calculated by dividing a difference between the transverse cross-sectional area of the base end portion of the tapered area 34 and the transverse cross-sectional area of the leading end portion of the tapered area 34 by the length L2 of the tapered area 34 in the axial line direction. Specifically, the transverse cross-sectional area increase ratio of the tapered area 34 is calculated by dividing the difference between the maximum transverse cross-sectional area and the minimum transverse cross-sectional area of the tapered area 34 by the length L2 of the tapered area 34 in the axial line direction. Similarly, the transverse cross-sectional area increase ratio of the tapered area 35 is calculated by dividing a difference between the transverse cross-sectional area of the base end portion of the tapered area 35 and the transverse cross-sectional area of the leading end portion of the tapered area 35 by the length L3 of the tapered area 35 in the axial line direction. Specifically, the transverse cross-sectional area increase ratio of the tapered area 35 is calculated by dividing the difference between the maximum transverse cross-sectional area and the minimum transverse cross-sectional area of the tapered area 35 by the length L3 of the tapered area 35 in the axial line direction. Further, the difference between the maximum transverse cross-sectional area and the minimum transverse cross-sectional area of the tapered areas 34 and 35 is the variation amount of the transverse cross-sectional area in each of the areas.

As described above, the rate of increase of the transverse cross-sectional area of the first tapered area 34 is larger than the rate of increase of the transverse cross-sectional area of the second tapered area 35. Thus, when the transverse cross-sectional area increase ratio of the first tapered area 34 is ΔS1, and the transverse cross-sectional area increase ratio of the second tapered area 35 is ΔS2, ΔS1 > ΔS2 is obtained. Further, the base end side area 33 that is continuous toward the base end side from the second tapered area 35 is the non-tapered area. Thus, when the transverse cross-sectional area increase ratio of the base end side area 33 is ΔS3, ΔS3 = 0 is obtained. As a result, magnitude relationships of the transverse cross-sectional area increase ratios ΔS1 to ΔS3 of each of the first tapered area 34, the second tapered area 35, and the base end side area 33 are: ΔS1 > ΔS2 > ΔS3.

In the present embodiment, the difference between the transverse cross-sectional area increase ratio ΔS1 of the first tapered area 34 and the transverse cross-sectional area increase ratio ΔS2 of the second tapered area 35 is the same or substantially the same as the difference between the transverse cross-sectional area increase ratio ΔS2 of the second tapered area 35 and the transverse cross-sectional area increase ratio ΔS3 of the base end side area 33. Thus, in the core wire 30, change amounts (the variation amounts) in the transverse cross-sectional area increase ratio of each of the boundary portion 36 between the first tapered area 34 and the second tapered area 35 and a boundary portion 37 between the second tapered area 35 and the base end side area 33 are the same.

As shown in Fig. 2, the core wire 30 is inserted through the outer tube hole 15a of the outer shaft 15 and is disposed across the guide wire port 21 in the axial line direction. Specifically, a leading end of the core wire 30 is disposed further to the leading end side than the guide wire port 21. Abase end of the core wire 30 is positioned further to the base end side than the guide wire port 21. Although not shown in the drawings, a base end portion of the core wire 30 is fixed to the hub 12.

In a state in which the core wire 30 is disposed in this way, the leading end side area 31 is disposed further to the leading end side than the guide wire port 21 and is inserted between the outer peripheral surface of the inner shaft 16 and the inner peripheral surface of the outer shaft 15. Further, the first tapered area 34 that is continuous toward the base end side from the leading end side area 31 is disposed such that it straddles the guide wire port 21 in the axial line direction. The first tapered area 34 is disposed such that a position of a portion of the first tapered area 34 on the leading end side is the same as the position of the guide wire port 21. The base end of the first tapered area 34 is positioned further to the base end side than the guide wire port 21.

As described above, the rate of increase, from the leading end side toward the base end side, of the outer diameter (the transverse cross-sectional area) of the first tapered area 34, which is on the leading end side of the tapered area 32, is greater than that of the second tapered area 35, which is on the base end side. Thus, in comparison to a configuration in which the outer diameter (the transverse cross-sectional area) of the tapered area increases at a constant rate from the leading end side toward the base end side, it is possible to make the leading end side outer diameter of the tapered area 32 larger. Accordingly, the outer diameter (the transverse cross-sectional area) of the core wire 30 in a position in the vicinity on the base end side of the guide wire port 21 in the axial line direction can be made larger. As a result, it is possible to increase the rigidity of the core wire 30.

Next, a method of use of the balloon catheter 10 will be briefly explained.

The user first inserts a guiding catheter through a sheath introducer that has been inserted into a blood vessel, and introduces a leading end opening portion of the guiding catheter as far as a coronary ostium. The user inserts the guide wire G through the guiding catheter and introduces the inserted guide wire G from the coronary ostium as far as a peripheral site via a portion to be treated, such as a constricted portion.

Next, the user inserts the guide wire G through the inner tube hole 16a of the balloon catheter 10. Then, in the state in which the guide wire G is inserted through the inner tube hole 16a, the user introduces the balloon catheter 10 into the human body while applying a push and pull maneuver while aligning the balloon catheter 10 with the guide wire G. The user disposes the balloon 13 in the portion to be treated inside the human body.

In the state in which the guide wire G is inserted through the inner tube hole 16a, it is conceivable that the rigidity of the area further to the leading end side than the guide wire port 21, through which the guide wire G is inserted, is higher than the rigidity of the area further to the base end side than the guide wire port 21, through which the guide wire G is not inserted. In this case, at the formation location of the guide wire port 21 in the axial line direction, the rigidity of the balloon catheter 10 changes locally. Since the rigidity of the balloon catheter 10 changes locally in the axial line direction, it is possible that a force pressing the balloon catheter 10 from the base end side is not properly transmitted to the leading end side.

In the present embodiment, as described above, the tapered area 32 of the core wire 30 has the two areas 34 and 35 that have mutually different rates of increase of the transverse cross-sectional area. Accordingly, by making the transverse cross-sectional area of the core wire 30 in the position in the vicinity on the base end side of the guide wire port 21 larger, rigidity in the vicinity on the base end side of the guide wire port 21 is increased. Therefore, it is possible to suppress the local changes in rigidity at the formation location of the guide wire port 21. As a result, it is possible to increase the transmissibility of the pressing force of the balloon catheter 10. In this way, it is possible to improve operability when introducing the balloon catheter 10 into the human body.

After disposing the balloon 13 in the portion to be treated, the user supplies the compressed fluid to the balloon 13 via the outer tube hole 15a of the outer shaft 15, from the hub 12 side, using a pressurizer. The balloon 13 is thus inflated. When the balloon 13 inflates, the constricted portion is expanded.

According to the configuration of the present embodiment described in detail above, the following excellent effects are obtained.

When the rate of increase of the transverse cross-sectional area from the leading end side toward the base end side in the first tapered area 34 is compared between a first configuration in which the boundary portion 36 between the first tapered area 34 and the second tapered area 35 is positioned to the leading end side in the tapered area 32, and a second configuration in which the boundary portion 36 between the first tapered area 34 and the second tapered area 35 is positioned to the base end side in the tapered area 32, it is conceivable that the rate of increase is larger in the first configuration than in the second configuration. Note that the transverse cross-sectional area of the boundary portion 36 in the first configuration and the transverse cross-sectional area of the boundary portion 36 in the second configuration are the same. Taking this point into account, in the present embodiment, the boundary portion 36 is positioned further to the leading end side than the center position of the tapered area 32 in the axial line direction. In this way, by making the rate of increase of the transverse cross-sectional area in the first tapered area 34 larger, it is possible to enlarge the transverse cross-sectional area of the core wire 30 in the position in the vicinity on the base end side of the guide wire port 21 in the axial line direction. As a result, the rigidity of the core wire 30 can be enhanced. Thus, it is possible to improve the transmissibility of the pressing force when introducing the balloon catheter 10 into the human body.

Specifically, the boundary portion 36 is positioned on the leading end side of the location that is positioned to the base end side by a distance of 1/4 of the total length (L2 + L3) of the tapered area 32 from the leading end portion of the tapered area 32. Thus, the rate of increase of the transverse cross-sectional area in the first tapered area 34 can be made even larger. As a result, it is possible to further enlarge the transverse cross-sectional area of the core wire 30 in the position in the vicinity on the base end side of the guide wire port 21 in the axial line direction. Accordingly, the rigidity of the core wire 30 can be further enhanced. Thus, it is possible to further improve the transmissibility of the pressing force when introducing the balloon catheter 10 into the human body.
Moreover, in the present embodiment, the boundary portion 36 is positioned further to the leading end side than the center position of the tapered area 32, and the transverse cross-sectional area of the boundary portion 36 is larger than a mean value of the transverse cross-sectional areas of both end portions of the tapered area 32 in the axial line direction. According to this configuration, compared to a configuration in which the transverse cross-sectional area of the boundary portion 36 is equal to or less than the mean value of the transverse cross-sectional areas of both end portions of the tapered area 32 in the axial line direction, it is possible to make the rate at which the transverse cross-sectional area of the first tapered area 34 increases from the leading end side toward the base end side larger. As a result, it is possible to even further enlarge the transverse cross-sectional area of the core wire 30 in the position in the vicinity on the base end side of the guide wire port 21 in the axial line direction. Thus, the rigidity of the core wire 30 can be even further enhanced. Accordingly, it is possible to even further improve the transmissibility of the pressing force when introducing the balloon catheter 10 into the human body.

The difference between the transverse cross-sectional area increase ratio ΔS1 of the first tapered area 34 and the transverse cross-sectional area increase ratio ΔS2 of the second tapered area 35 is the same as the difference between the transverse cross-sectional area increase ratio ΔS2 of the second tapered area 35 and the transverse cross-sectional area increase ratio ΔS3 of the base end side area 33. Accordingly, changes (amounts) that occur locally in the rate of increase of the transverse cross-sectional area in the boundary portion 36 between the first tapered area 34 and the second tapered area 35 and the boundary portion 37 between the second tapered area 35 and the base end side area 33, respectively, can be made the same. In other words, in this case, local changes in the rate of increase of the transverse cross-sectional area occurring respectively in each of the boundary portions 36 and 37 can be uniformly distributed to each of the boundary portions 36 and 37. As a result, it is possible to suppress the local changes occurring respectively in each of the boundary portions 36 and 37. It is thus possible to suppress local changes in rigidity occurring respectively in each of the boundary portions 36 and 37. Consequently, with the configuration in which the two tapered areas 34 and 35 having the different rates of increase of the transverse cross-sectional area are provided in the tapered area 32, it is possible to suppress a deterioration in the kink resistance performance.

The core wire 30 is provided in the outer tube hole 15a of the outer shaft 15 of the balloon catheter 10. The leading end side area 31 of the core wire 30 is inserted between the inner peripheral surface of the outer shaft 15 and the outer peripheral surface of the inner shaft 16. The compressed fluid flows between the inner peripheral surface of the outer shaft 15 and the outer peripheral surface of the inner shaft 16. Thus, in order to suppress a deterioration in flowability of the fluid, it is necessary to make the transverse cross-sectional area of the leading end side area 31 as small as possible. According to this, there is a possibility that, in the tapered area 32 extending from the leading end side area 31 to the base end side also, the transverse cross-sectional area in the position in the vicinity on the base end side of the guide wire port 21 may also become smaller. In the balloon catheter 10 provided with the core wire 30 of the present embodiment, it is possible to enlarge the transverse cross-sectional area of the core wire 30 in the position in the vicinity on the base end side of the guide wire port 21. By enlarging the transverse cross-sectional area of the core wire 30 in the position in the vicinity on the base end side of the guide wire port 21, a through-flow performance of the compressed fluid is secured, and it is possible to yet further improve the transmissibility of the pressing force when introducing the balloon catheter 10 into the human body.

In the above-described embodiment, the outer shaft 15 corresponds to an "outer tube" and a "tube." The inner shaft 16 corresponds to "an inner tube." The outer tube hole 15a corresponds to a "lumen." The present invention is not limited to the above-described embodiment, and may be executed in the following manner, for example.
(1) In the above-described embodiment, the dimension ratio of the length L2 of the first tapered area 34 and the length L3 of the second tapered area 35 L2/L3 is smaller than 1/5. However, L2/L3 may be a numeral value that is equal to or greater than 1/5 and smaller than 1. Even in this case, since the boundary portion 36 between the first tapered area 34 and the second tapered area 35 is positioned further to the leading end side than the center position of the tapered area 32 in the axial line direction, in comparison to a case in which the boundary portion 36 is positioned further to the base end side than the center position of the tapered area 32, it is possible to make larger the rate of increase of the outer diameter (the transverse cross-sectional area) from the base end side toward the leading end side in the first tapered area 34. Thus, it is possible to enlarge the outer diameter of the core wire 30 in the position in the vicinity on the base end side of the guide wire port 21 in the axial line direction. It is therefore possible to increase the rigidity of the core wire 30. As a result, in this case also, it is possible to improve the transmissibility of the pressing force when introducing the balloon catheter 10 into the human body.
   It should be noted that the boundary portion 36 may be positioned further to the base end side than the center position of the tapered area 32 in the axial line direction. In other words, L2/L3 may be equal to or greater than 1.
(2) In the above-described embodiment, the base end side area 33 is formed as a non-tapered shape with a constant outer diameter (transverse cross-sectional area) across the whole area in the axial line direction. However, the base end side area 33 may be formed in a tapered shape whose outer diameter (transverse cross-sectional area) becomes larger from the base end side toward the leading end side. In this case, the rate of increase of the transverse cross-sectional area from the base end side toward the leading end side in the base end side area 33 (the transverse cross-sectional area increase ratio ΔS3) may be smaller than the rate of increase of the transverse cross-sectional area in the second tapered area 35. In this configuration also, if the difference between the transverse cross-sectional area increase ratio ΔS2 of the second tapered area 35 and the transverse cross-sectional area increase ratio ΔS3 of the base end side area 33 is the same as the difference between the transverse cross-sectional area increase ratio ΔS1 of the first tapered area 34 and the transverse cross-sectional area increase ratio ΔS2 of the second tapered area 35, local change amounts of the transverse cross-sectional area in the boundary portion 37 between the second tapered area 35 and the base end side area 33, and in the boundary portion 36 between the first tapered area 34 and the second tapered area 35, respectively, become the same. Specifically, it is possible to cause amounts of local changes in rigidity of the core wire 30 to be the same. As a result, in the configuration in which the tapered area 32 has the two areas 34 and 35 with the mutually different rates of increase of the transverse cross-sectional area, it is possible to suppress a deterioration in the kink resistance performance.
   Further, the core wire 30 need not necessarily be provided with the base end side area 33. In this case, it is sufficient that the second tapered area 35 extends continuously as far as the base end portion of the core wire 30.
(3) In the above-described embodiment, the difference between the transverse cross-sectional area increase ratio ΔS1 of the first tapered area 34 and the transverse cross-sectional area increase ratio ΔS2 of the second tapered area 35 is the same as the difference between the transverse cross-sectional area increase ratio ΔS2 of the second tapered area 35 and the transverse cross-sectional area increase ratio ΔS3 of the base end side area 33 (ΔS1 - ΔS2 = ΔS2 - ΔS3). However, it is possible to be ΔS1 - ΔS2 > ΔS2 - ΔS3. Alternatively, it is possible to be ΔS1 - ΔS2 < ΔS2 - ΔS3.
(4) In the above-described embodiment, the transverse cross-sectional area of the boundary portion 36 between the first tapered area 34 and the second tapered area 35 is larger than the mean value of the transverse cross-sectional area of the leading end portion of the tapered area 32 and the transverse cross-sectional area of the base end portion of the tapered area 32. However, the transverse cross-sectional area of the boundary portion 36 between the first tapered area 34 and the second tapered area 35 may be the same as or smaller than the mean value of the transverse cross-sectional area of the leading end portion of the tapered area 32 and the transverse cross-sectional area of the base end portion of the tapered area 32.
(5) In the above-described embodiment, the first tapered area 34 is positioned such that the leading end side of the first tapered area 34 is in the same position as the guide wire port 21 in the axial line direction. However, the first tapered area 34 may be positioned such that the base end side of the first tapered area 34 is in the same position as the guide wire port 21 in the axial line direction. Alternatively, the first tapered area 34 may be positioned such that a center portion of the first tapered area 34 is in the same position as the guide wire port 21 in the axial line direction. Moreover, the first tapered area 34 need not necessarily be positioned across the guide wire port 21 in the axial line direction. For example, the first tapered area 34 may be positioned such that the leading end of the first tapered area 34 is in the same position as the guide wire port 21 in the axial line direction. In this way, it is sufficient that the first tapered area 34 includes the same position as the guide wire port 21 in the axial line direction.
(6) In the above-described embodiment, the example is explained in which the present invention is applied to the balloon catheter. However, the present invention may be applied to another Rx type catheter in which the guide wire port 21 is formed in the midway position in the axial line direction. The present invention may be applied to a catheter that is not provided with a balloon. Moreover, in the above-described embodiment, the catheter shaft 11 includes the plurality of tube shafts. However, the catheter shaft need not necessarily include the plurality of tube shafts. Specifically, the present invention may be applied to a catheter provided with only a single shaft. In this case, for example, it is sufficient that the single shaft have a lumen in the interior thereof. It is sufficient that the core wire be inserted into the lumen. In this case, it is sufficient that a guide wire port, which penetrates an outer peripheral wall surrounding the lumen of the shaft, be formed in a midway position in the axial line direction of the single shaft. Further, the single shaft may have two lumens in the interior thereof. In this case, of the two lumens, one of the lumens may be a lumen through which a guide wire can be inserted and the other lumen may be a lumen through which compressed fluid can flow. It is sufficient that the guide wire port be formed in the lumen through which the guide wire can be inserted, in a midway position in the axial line direction, such that the guide wire port penetrates the lumen. Furthermore, the single shaft may have three or more lumens.

### Description of Reference Numerals

10:Balloon catheter, 13:Balloon, 15:Outer tube as tube, 15a:Outer tube hole as lumen and fluid lumen, 16:Inner tube, 16a:Inner tube hole as guide wire lumen, 21:Guide wire port, 23:Peripheral wall portion, 30:Core wire, 31:Leading end side area, 32:Tapered area, 33:Base end side area, 34:First tapered area, 35:Second tapered area, 36:Boundary portion

## Claims

1. A catheter (10) comprising:
a tube (15) having a lumen (15a) in an interior thereof and a peripheral wall portion (23) surrounding the lumen;
a guide wire port (21) formed in a midway position in an axial line direction of the tube, the guide wire port penetrating the peripheral wall portion; and
a core wire (30) inserted into the lumen and extending across the guide wire port in the axial line direction;
and **characterized in that**,
the core wire includes
a leading end side area (31), which is an area positioned further to a leading end side than the guide wire port, and
a tapered area (32), which is an area further to a base end side than the leading end side area and which is formed such that a cross-sectional area thereof in a direction orthogonal to the axial line direction becomes continuously larger from the leading end side toward the base end side,
the tapered area includes
a first tapered area (34), which is an area extending from a base end of the leading end side area toward the base end side, and which is an area including a same position as the guide wire port in the axial line direction, and
a second tapered area (35), which is an area extending from a base end of the first tapered area toward the base end side, and
a rate of increase of the cross-sectional area from the leading end side toward the base end side in the first tapered area is larger than a rate of increase of the cross-sectional area from the leading end side toward the base end side in the second tapered area.

2. The catheter according to claim 1, **characterized in that**
a boundary portion (36) between the first tapered area and the second tapered area is positioned further to the leading end side than a center of the tapered area in the axial line direction.

3. The catheter according to claim 2, **characterized in that**
the boundary portion is positioned further to the leading end side than a location positioned to the base end side, from a leading end portion of the tapered area, by a distance of 1/4 of a total length of the tapered area.

4. The catheter according to claim 2 or 3, **characterized in that**
the cross-sectional area of the boundary portion is larger than a mean value of the cross-sectional areas of both ends of the tapered area in the axial line direction.

5. The catheter according to any one of claims 1 to 4, **characterized in that**
the core wire further includes a base end side area (33), which is an area extending from a base end of the second tapered area toward the base end side,
the base end side area is formed to have the cross-sectional area that is constant from the leading end side toward the base end side, or is formed to have the cross-sectional area that becomes larger from the leading end side toward the base end side at a rate of increase that is smaller than the second tapered area, and
a difference between the rates of increase of the cross-sectional area of the first tapered area and the second tapered area is the same as or substantially the same as a difference between the rates of increase of the cross-sectional area of the second tapered area and the base end side area.

6. The catheter according to any one of claims 1 to 5, **characterized by** comprising:
an outer tube (15) corresponding to the tube, the outer tube internally having a fluid lumen (15a) corresponding to the lumen;
an inner tube (16) inserted into the fluid lumen in a state in which a part of the inner tube extends further to the leading end side than a leading end of the outer tube, the inner tube having a guide wire lumen (16a) through which the guide wire is insertable, and a base end portion of which communicates with the guide wire port; and
a balloon (13), which covers, from the outside, the part of the inner tube extending from the leading end of the outer tube, and a base end portion of which is connected to a leading end portion of the outer tube, wherein
the leading end side area of the core wire is inserted into the fluid lumen between an outer peripheral surface of the inner tube and an inner peripheral surface of the outer tube.

## Patentansprüche

1. Katheter (10), umfassend:
ein Rohr (15) mit einem Lumen (15a) darin und einem Außenwandteil (23), der das Lumen umgibt;
eine Führungsdrahtöffnung (21), ausgebildet an einer mittleren Stelle in einer Axiallinienrichtung des Rohrs, wobei die Führungsdrahtöffnung den Außenwandteil durchdringt; und
einen Kerndraht (30), eingesetzt in das Lumen und sich an der Führungsdrahtöffnung vorbei in der Axiallinienrichtung erstreckend;
und **dadurch gekennzeichnet, dass**:
der Kerndraht enthält:
einen vorderendseitigen Bereich (31), der ein Bereich ist, der weiter zu einer Vorderendseite liegt als die Führungsdrahtöffnung, und
einen verjüngten Bereich (32), der ein Bereich ist, der weiter zu einer Basisendseite liegt als die vorderendseitige Bereich, und der so ausgebildet ist, dass eine Querschnittsfläche davon in einer Richtung senkrecht zur Axiallinienrichtung von der Vorderendseite zur Basisendseite hin fortlaufend größer wird,
wobei der verjüngte Bereich enthält:
einen ersten verjüngten Bereich (34), der sich von einem Basisende des vorderendseitigen Bereichs zur Basisendseite hin erstreckt, und der ein Bereich ist, der in der Axiallinienrichtung eine selbe Stelle enthält wie die Führungsdrahtöffnung, und
einen zweiten verjüngten Bereich (35), der sich von einem Basisende des ersten verjüngten Bereichs zur Basisendseite hin erstreckt, und
eine Vergrößerungsrate der Querschnittsfläche von der Vorderendseite zur Basisendseite hin im ersten verjüngten Bereich größer ist als eine Vergrößerungsrate der Querschnittsfläche von der Vorderendseite zur Basisendseite hin im zweiten verjüngten Bereich.

2. Katheter nach Anspruch 1, **dadurch gekennzeichnet, dass**
ein Grenzteil (36) zwischen dem ersten verjüngten Bereich und dem zweiten verjüngten Bereich weiter zur Vorderendseite liegt als eine Mitte des verjüngten Bereichs in der Axiallinienrichtung.

3. Katheter nach Anspruch 2, **dadurch gekennzeichnet, dass**
der Grenzteil weiter zur Vorderendseite liegt als eine Stelle, die zur Basisendseite liegt, von einem Vorderendteil des verjüngten Bereichs, um eine Strecke von 1/4 einer Gesamtlänge des verjüngten Bereichs.

4. Katheter nach Anspruch 2 oder 3, **dadurch gekennzeichnet, dass**
die Querschnittsfläche des Grenzteils größer ist als ein Mittelwert der Querschnittsflächen beider Enden des verjüngten Bereichs in der Axiallinienrichtung.

5. Katheter nach einem beliebigen der Ansprüche 1 bis 4, **dadurch gekennzeichnet, dass**
der Kerndraht weiter einen basisendseitigen Bereich (33) enthält, der ein Bereich ist, der sich von einem Basisende des zweiten verjüngten Bereichs zur Basisendseite hin erstreckt,
der basisendseitige Bereich so ausgebildet ist, dass er die Querschnittsfläche aufweist, die konstant ist von der Vorderendseite zur Basisendseite hin, oder so ausgebildet ist, dass er die Querschnittsfläche aufweist, die von der Vorderendseite zur Basisendseite hin mit einer Vergrößerungsrate größer wird, die kleiner ist als beim zweiten verjüngten Bereich, und
eine Differenz zwischen den Vergrößerungsraten der Querschnittsfläche des ersten verjüngten Bereichs und des zweiten verjüngten Bereichs dieselbe oder im Wesentlichen dieselbe ist wie eine Differenz zwischen den Vergrößerungsraten der Querschnittsfläche des zweiten verjüngten Bereichs und des basisendseitigen Bereichs.

6. Katheter nach einem beliebigen der Ansprüche 1 bis 5, **dadurch gekennzeichnet, dass** er umfasst:
ein Außenrohr (15), das dem Rohr entspricht, wobei das Außenrohr innen ein Fluidlumen (15a) aufweist, das dem Lumen entspricht;
ein in das Fluidlumen in einem Zustand eingesetztes Innenrohr (16), in dem sich ein Teil des Innenrohrs weiter zur Vorderendseite erstreckt als ein Vorderende des Außenrohrs, wobei das Innenrohr ein Führungsdrahtlumen (16a) aufweist, durch das der Führungsdraht einsetzbar ist, und von dem ein Basisendteil mit der Führungsdrahtöffnung in Verbindung steht; und
einen Ballon (13), der von außen den Teil des Innenrohrs bedeckt, der sich vom Vorderende des Außenrohrs erstreckt, und von dem ein Basisendteil mit einem Vorderendteil des Außenrohrs verbunden ist, wobei
der vorderendseitige Bereich des Kerndrahtes in das Fluidlumen zwischen einer Außenumfangsfläche des Innenrohrs und einer Innenumfangsfläche des Außenrohrs eingesetzt ist.

## Revendications

1. Cathéter (10) comprenant :
un tube (15) ayant un lumen (15a) en son intérieur et une partie de paroi périphérique (23) entourant le lumen ;
un orifice (21) de fil-guide formé à une position à mi-chemin dans une direction de ligne axiale du tube, l'orifice de fil-guide pénétrant dans la partie de paroi périphérique ; et
un fil d'âme (30) inséré dans le lumen et s'étendant à travers l'orifice de fil-guide dans la direction de ligne axiale ;
et **caractérisé en ce que**
le fil d'âme comprend
une zone latérale (31) d'extrémité avant, qui est une zone positionnée plus loin d'un côté d'extrémité avant que l'orifice de fil-guide, et
une zone conique (32) qui est une zone plus éloignée d'un côté d'extrémité de base que la zone latérale d'extrémité avant et qui est formée de telle sorte qu'une aire de section droite de celle-ci, dans une direction orthogonale à la direction de ligne axiale, devienne continuellement plus grande depuis le côté d'extrémité avant vers le côté d'extrémité de base,
la zone conique comprend
une première zone conique (34), qui est une zone s'étendant depuis une extrémité de base de la zone latérale d'extrémité avant vers le côté d'extrémité de base, et qui est une zone comprenant une même position que l'orifice de fil-guide dans la direction de ligne axiale, et
une seconde zone conique (35), qui est une zone s'étendant depuis une extrémité de base de la première zone conique vers le côté d'extrémité de base, et
un taux d'accroissement de l'aire de section droite depuis le côté d'extrémité avant vers le côté d'extrémité de base dans la première zone conique est plus grand qu'un taux d'accroissement de l'aire de section droite du côté d'extrémité avant vers le côté d'extrémité de base dans la seconde zone conique.

2. Cathéter selon la revendication 1, **caractérisé en ce que**
une partie limite (36) entre la première zone conique et la seconde zone conique est positionnée plus loin du côté d'extrémité avant qu'un centre de la zone conique dans la direction de ligne axiale.

3. Cathéter selon la revendication 2, **caractérisé en ce que**
la partie limite est positionnée plus loin du côté d'extrémité avant qu'un emplacement positionné du côté d'extrémité de base, à partir d'une partie d'extrémité avant de la zone conique, d'une distance de 1/4 d'une longueur totale de la zone conique.

4. Cathéter selon la revendication 2 ou 3, **caractérisé en ce que**
l'aire de la section droite de la partie limite est plus grande qu'une valeur moyenne des aires de section droite des deux extrémités de la zone conique dans la direction de ligne axiale.

5. Cathéter selon l'une quelconque des revendications 1 à 4, **caractérisé en ce que**
le fil d'âme comprend en outre une zone latérale (33) d'extrémité de base, qui est une zone s'étendant depuis une extrémité de base de la seconde zone conique vers le côté d'extrémité de base,
la zone latérale d'extrémité de base est formée de manière à avoir une aire de section droite qui est constante depuis le côté d'extrémité avant vers le côté d'extrémité de base, ou est formée de manière à avoir une aire de section droite qui augmente depuis le côté d'extrémité avant vers le côté d'extrémité de base à un taux d'accroissement qui est plus faible que la seconde zone conique, et
une différence entre les taux d'accroissement de l'aire de section droite de la première zone conique et de la seconde zone conique est identique ou sensiblement identique à une différence entre les taux d'accroissement de l'aire de section droite de la seconde zone conique et de la zone latérale d'extrémité de base.

6. Cathéter selon l'une quelconque des revendications 1 à 5, **caractérisé en ce qu'**il comprend :
un tube extérieur (15) correspondant au tube, le tube extérieur ayant intérieurement un lumen (15a) pour fluide correspondant au lumen ;
un tube intérieur (16) inséré dans le lumen pour fluide dans un état dans lequel une partie du tube intérieur s'étend plus loin du côté d'extrémité avant qu'une extrémité avant du tube extérieur, le tube intérieur ayant un lumen (16a) de fil-guide à travers lequel le fil-guide peut être inséré, et dont une partie d'extrémité de base communique avec l'orifice de fil-guide ; et
un ballon (13) qui couvre, depuis l'extérieur, la partie du tube intérieur s'étendant depuis l'extrémité avant du tube extérieur, et dont une partie d'extrémité de base est reliée à une partie d'extrémité avant du tube extérieur, dans lequel
la zone latérale d'extrémité avant du fil d'âme est insérée dans le lumen pour fluide entre une surface périphérique extérieure du tube intérieur et une surface périphérique intérieure du tube extérieur.
